# EUROPEAN PATENT APPLICATION

(11) **EP 2 554 671 A1**
(43) Date of publication of application: **06.02.2013**
(21) Application number: 11765685.0
(22) Date of filing: 30.03.2011
(51) Int. Cl.: C12N 15/09, C12N 1/15, C12N 1/19, C12N 1/21, C12N 5/10, C12N 9/88

(54) **NOVEL ALLENE OXIDE SYNTHASE DERIVED FROM LEMNA PAUCICOSTATA**

(30) Priority: 31.03.2010 JP 2010082354
(71) Applicant: Shiseido Company, Ltd., Chuo-ku Tokyo 104-8010 (JP)
(72) Inventor: YOKOYAMA, Mineyuki, Yokohama-shi Kanagawa 236-8643 (JP); KAMICHI, Sari, Yokohama-shi Kanagawa 236-8643 (JP); TAKAGI, Kazuteru, Yokohama-shi Kanagawa 236-8643 (JP)
(74) Representative: Henkel, Breuer & Partner
(86) International application number: PCT/JP2011/058108
(87) International publication number: WO 2011/125785

(57) **Abstract**

Disclosed is a high-activity allene oxide synthase that can be used in the production of a plant growth regulator (KODA).

SEQUENCE LISTING

<110>  SHISEIDO COMPANY, LTD.

<120>  Novel Allene Oxide Synthase derived from Lemna paucicostata

<130>  Z570-PCT

<150>  JP2010-082354
<151>  2010-03-31

<160>  8

<170>  PatentIn version 3.3

<210>  1
<211>  1443
<212>  DNA
<213>  Lemna paucicostata

<400>  1
atgtctgtct cgcaatcaga tgacaaggcc gccctggtgc cgcagaagac catcccaggg      60

agctatggca tacctttcat cactcccctc aaggatcggc tcgacttctt cagcaacgaa     120

taccaattct tccagtctcg cgtagaaagc tatggctcca ccatcgtccg cctgaacgct     180

ccgccaggac ccttcatggc gaaaaaccct caagtcatcg ccattctcga cggcaagagc     240

ttccccgtcc tcttcgacac ctccaaggtc gagaaaaaga acatcttcac cggcacttac     300

atgccgtcta ctgcactcac cggcggctac cgagtttgcg cttatctcga tccgtcggag     360

cccaaccaca caaaaatcaa gcaacttctc ctgaacatcc tcttcaacag aaaagaccat     420

gtaatcccgg agttccaccg cgcgtacgaa aagctcttcg acgatatgga cgcggaaatc     480

gccaaatctg gaaaatttgt ctttaatgac cacaacgacg gtgcagcgtt cgagtttctg     540

ggcagactct tctttggagt gagcccttcg gagacggagc ttggagccgg aggagttaag     600

gacgccaatt tatggctatt ttcgcagctc tgccccctca tgaccctcgg ctttttgcct     660

aagttattgg aagatctcct cctgcacact tttcccttgc cacccttcct tttcaagggt     720

aagtaccagg cgatttacaa gtatatcagc tccgtcgcga cggacgccct aactatggcc     780

gagaatctcg ggctctcccg ggaagaagct gcccataacc tcctcttcgc cgtgtgcttt     840

aactcgctgg gcggcgtgaa agtcctcttt cccggaattc tcagatatat agcgcaagcc     900

gggaaaaatc tccaagccag ccttgtaagc gaggttcgtt ccgccgtgag ctcaactggc     960

ggggagttga cgattgaagc cctggagaag atgccgctga ccaagtcagt ggtctacgag    1020

tccctccgcc tcgatcctcc cgtcaagtac cagtacggcg tggtgaagaa ggacatggtc    1080

atcgagagcc atgacaggag ctacgaggta aaggccgggg aaacgttatt cggctaccag    1140

cctttcgcca cgagggacaa gaagattttt gggccggacg cggacaactt cgtggcggag    1200

aggttcatcg gagaagaagg cgctaaagtg ttgcggttcg ttgtgtggtc caatgggccg    1260

gagacaacag accctacgcc ggtcgataag caatgccccg ggaagaacct ggtcgtgctg    1320

atttctcggc tgctggtggc ggagtttttc ctgcgctacg acttcttcga cgcggagata 1380

ggagccgtgc cgcttgccgt caaaaccacc atcacctcct tgacgaaggc gacgacccga 1440

taa 1443

<210> 2
<211> 480
<212> PRT
<213> Lemna paucicostata

<400> 2

Met Ser Val Ser Gln Ser Asp Asp Lys Ala Ala Leu Val Pro Gln Lys
1               5                   10                  15

Thr Ile Pro Gly Ser Tyr Gly Ile Pro Phe Ile Thr Pro Leu Lys Asp
            20                  25                  30

Arg Leu Asp Phe Phe Ser Asn Glu Tyr Gln Phe Phe Gln Ser Arg Val
        35                  40                  45

Glu Ser Tyr Gly Ser Thr Ile Val Arg Leu Asn Ala Pro Pro Gly Pro
    50                  55                  60

Phe Met Ala Lys Asn Pro Gln Val Ile Ala Ile Leu Asp Gly Lys Ser
65                  70                  75                  80

Phe Pro Val Leu Phe Asp Thr Ser Lys Val Glu Lys Lys Asn Ile Phe
                85                  90                  95

Thr Gly Thr Tyr Met Pro Ser Thr Ala Leu Thr Gly Gly Tyr Arg Val
            100                 105                 110

Cys Ala Tyr Leu Asp Pro Ser Glu Pro Asn His Thr Lys Ile Lys Gln
        115                 120                 125

Leu Leu Leu Asn Ile Leu Phe Asn Arg Lys Asp His Val Ile Pro Glu
    130                 135                 140

Phe His Arg Ala Tyr Glu Lys Leu Phe Asp Asp Met Asp Ala Glu Ile
145                 150                 155                 160

Ala Lys Ser Gly Lys Phe Val Phe Asn Asp His Asn Asp Gly Ala Ala
                165                 170                 175

Phe Glu Phe Leu Gly Arg Leu Phe Phe Gly Val Ser Pro Ser Glu Thr
            180                 185                 190

Glu Leu Gly Ala Gly Gly Val Lys Asp Ala Asn Leu Trp Leu Phe Ser
        195                 200                 205

Gln Leu Cys Pro Leu Met Thr Leu Gly Phe Leu Pro Lys Leu Leu Glu
    210                 215                 220

Asp Leu Leu Leu His Thr Phe Pro Leu Pro Pro Phe Leu Phe Lys Gly
225                 230                 235                 240

Lys Tyr Gln Ala Ile Tyr Lys Tyr Ile Ser Ser Val Ala Thr Asp Ala
                245                 250                 255

Leu Thr Met Ala Glu Asn Leu Gly Leu Ser Arg Glu Glu Ala Ala His
            260                 265                 270

Asn Leu Leu Phe Ala Val Cys Phe Asn Ser Leu Gly Gly Val Lys Val
        275                 280                 285

Leu Phe Pro Gly Ile Leu Arg Tyr Ile Ala Gln Ala Gly Lys Asn Leu
    290                 295                 300

Gln Ala Ser Leu Val Ser Glu Val Arg Ser Ala Val Ser Ser Thr Gly
305                 310                 315                 320

Gly Glu Leu Thr Ile Glu Ala Leu Glu Lys Met Pro Leu Thr Lys Ser
                325                 330                 335

Val Val Tyr Glu Ser Leu Arg Leu Asp Pro Pro Val Lys Tyr Gln Tyr
            340                 345                 350

Gly Val Val Lys Lys Asp Met Val Ile Glu Ser His Asp Arg Ser Tyr
        355                 360                 365

Glu Val Lys Ala Gly Glu Thr Leu Phe Gly Tyr Gln Pro Phe Ala Thr
    370                 375                 380

Arg Asp Lys Lys Ile Phe Gly Pro Asp Ala Asp Asn Phe Val Ala Glu
385                 390                 395                 400

Arg Phe Ile Gly Glu Glu Gly Ala Lys Val Leu Arg Phe Val Val Trp
                405                 410                 415

Ser Asn Gly Pro Glu Thr Thr Asp Pro Thr Pro Val Asp Lys Gln Cys
            420                 425                 430

Pro Gly Lys Asn Leu Val Val Leu Ile Ser Arg Leu Leu Val Ala Glu
        435                 440                 445

Phe Phe Leu Arg Tyr Asp Phe Phe Asp Ala Glu Ile Gly Ala Val Pro
    450                 455                 460

Leu Ala Val Lys Thr Thr Ile Thr Ser Leu Thr Lys Ala Thr Thr Arg
465                 470                 475                 480

<210>  3
<211>  21
<212>  DNA
<213>  Artifical Sequence

<400>  3
ggaactaacc ggaggctacc g                                                21

<210>  4
<211>  25
<212>  DNA
<213>  Artifical Sequence

<400>  4
ccgtctccgg tccattcgac cacaa                                            25

<210>  5
<211>  23
<212>  DNA
<213>  Artifical Sequence

<400>  5
tctgggcaga ctcttctttg gag                                              23

<210>  6
<211>  27
<212>  DNA
<213>  Artifical Sequence

<400>  6
ccggcttgcg ctatatatct gagaatt                                          27

<210>  7
<211>  32
<212>  DNA
<213>  Artifical Sequence

<400>  7
ccgcgccatg gagaagagaa tgtctgtctc gc                                    32

<210>  8
<211>  30
<212>  DNA
<213>  Artifical Sequence

<400>  8
ccgcgctcga ggttgaataa gcacgagtgt                                       30

## Description

### [Technical Field]

The present invention relates to a novel allene oxide synthase gene derived from Lemna paucicostata, an expression product of said allene oxide synthase gene, and a method of producing allene oxide synthase using the allene oxide synthase gene.

### [Background Art]

Allene oxide synthase is an enzyme having an activity of converting a hydroperoxidated fatty acid to an allene oxide. Since said allene oxide is unstable, it is nonenzymatically converted to α-ketol form. Allene8 oxide synthase is known to present in plants, animals and yeast. Among the plants, it is known to be present in throughout the angiosperms. For example, it is recognized to present in barley, wheat, corn, cotton, egg plants, flax (seed etc.), lettuce, oat, spinach, sunflower etc. In plants, allene oxide synthase contributes to the pathway of jasmonic acid biosynthesis in response to stress such as pests and injuries, and is generally known to covert 13-hydroxyperoxy linolenic acid to 12,13-epoxylinolenic acid (Rika Ozawa, Protein, Nucleic acid and Enzyme, 48(13): 1786-1792, 2003)

The present inventors have found a α-ketol unsaturated fatty acid (hereinafter referred to as KODA) having a structure represented by the following Formula: 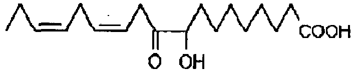 having a floral bud-formation promoting activity, a plant activation activity and a plant growth regulating activity comprising them (Japanese Unexamined Patent Publication (Kokai) No. 9-295908). KODA can be formed in a plant or enzymatically according to the following scheme: 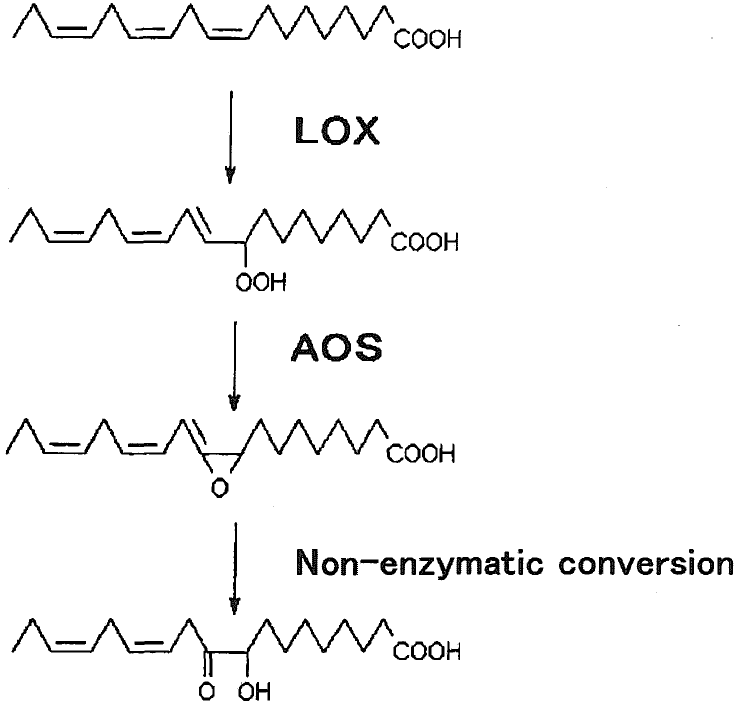 Thus, KODA is produced by reacting α-linolenic acid with position 9 product specific lipoxygenase (LOX) to obtain 9-hydroperoxylinolenic acid, and then reacting allene oxide synthase (AOS) to said 9-hydroperoxylinolenic acid, thereby hydroperoxy group at position 9 is dehydrated to form an epoxy group, and said epoxy compound is nonenzymatically converted to α-ketol form (Japanese Unexamined Patent Publication (Kokai) No. 11-29410, Japanese Unexamined Patent Publication (Kokai) No. 2001-131006, and Japanese Unexamined Patent Publication (Kokai) No. 2009-17829).

KODA was known to be present in a variety of plant species, and Lemna paucicostata that was subjected to a stress was known to release KODA at a very high level (a few hundred-fold higher) compared to other plants (Japanese Unexamined Patent Publication (Kokai) No. 11-29410). Thus, it was estimated that allene oxide synthase having an excellent activity compared to allene oxide synthases derived from other plants was present in Lemna paucicostata. However, cDNA of allene oxide synthase from Lemna paucicostata was not isolated and the sequence was unknown.

When KODA is to be produced by enzymatic synthesis using α-linolenic acid or linoleic acid as the starting substance, allene oxide synthase is required. However, allene oxide synthase generally has a suicide substrate-like property, and thus when the substrate concentration is increased, the amount of KODA produced conversely decreases, and thus it was difficult to produce KODA in large quantities by the enzymatic method. Thus, there has been a need for the identification of a highly active from of allene oxide synthase.

### [Summary of Invention]

### [Technical Problem]

The problem to be solved by the present invention is to identify allene oxide synthase useful for the formation of KODA, to isolate cDNA, to provide a vector having integrated said cDNA therein, to provide a transformant having introduced said vector therein, and to provide an expression product of the allene oxide synthase gene using said transformant, and even to provide a method of enzymatically producing compounds such as KODA and jasmonic acid by using said allene oxide synthase.

### [Solution to Problem]

In intensive research to solve the above problem, and to isolate a cDNA of novel allene oxide synthase derived from Lemna paucicostata, which releases a few hundred-fold more KODA than other plants, the present inventors conducted the screening of various strains of Lemna paucicostata for the ability of producing KODA in order to obtain the cDNA of allene oxide synthase having a higher activity. Specifically 62 strains of Lemna paucicostata were screened, and it was surprisingly found that Lemna paucicostata SH strain has a KODA producing activity 12-fold or more higher than the mean of other Lemna paucicostata strains.

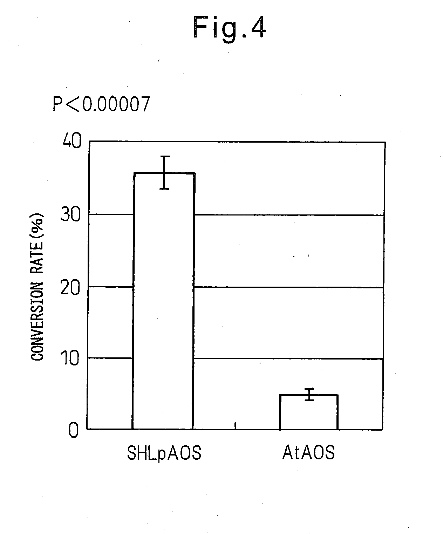

The present inventors have isolated allene oxide synthase cDNA from Lemna paucicostata SH strain, integrated it into a vector, introduced said vector into Escherichia coli to express the protein of allene oxide synthase, and examined the activity of said allene oxide synthase protein. It was revealed that allene oxide synthase derived from Lemna paucicostata SH strain had an activity higher than that of allene oxide synthase derived from Arabidopsis thaliana, which is conventionally used (Fig. 4).

Thus, the present inventors provide a novel allene oxide synthase gene derived from Lemna paucicostata SH strain and a protein encoded by said gene. Specifically, the present invention provides a gene consisting of a DNA having a base sequence of SEQ ID NO: 1, a gene consisting of a DNA substantially identical to said DNA, and proteins encoded by these genes. Furthermore, the present inventors provide an expression vector having the above DNA and a vector fragment bound to each other therein. Furthermore, the present invention also relates to a transformant of a microorganism, an animal cell or an insect cell transformed with said expression vector. The allene oxide synthase of the present invention can be obtained by culturing said transformant, recovering said transformant, extracting allene oxide synthase from said transformant, and purifying it.

### [Advantageous Effects of Invention]

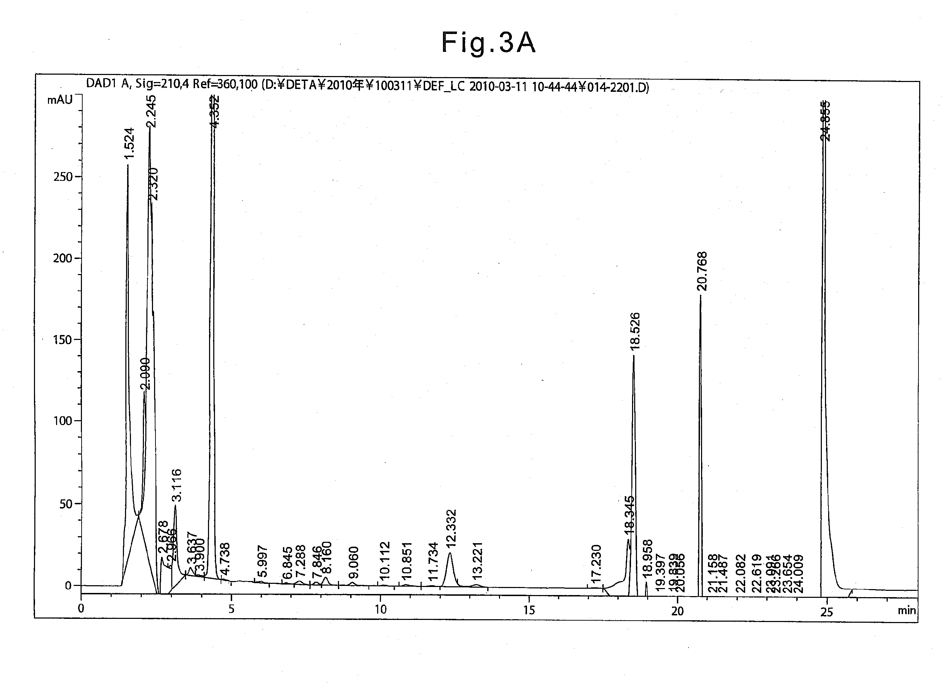

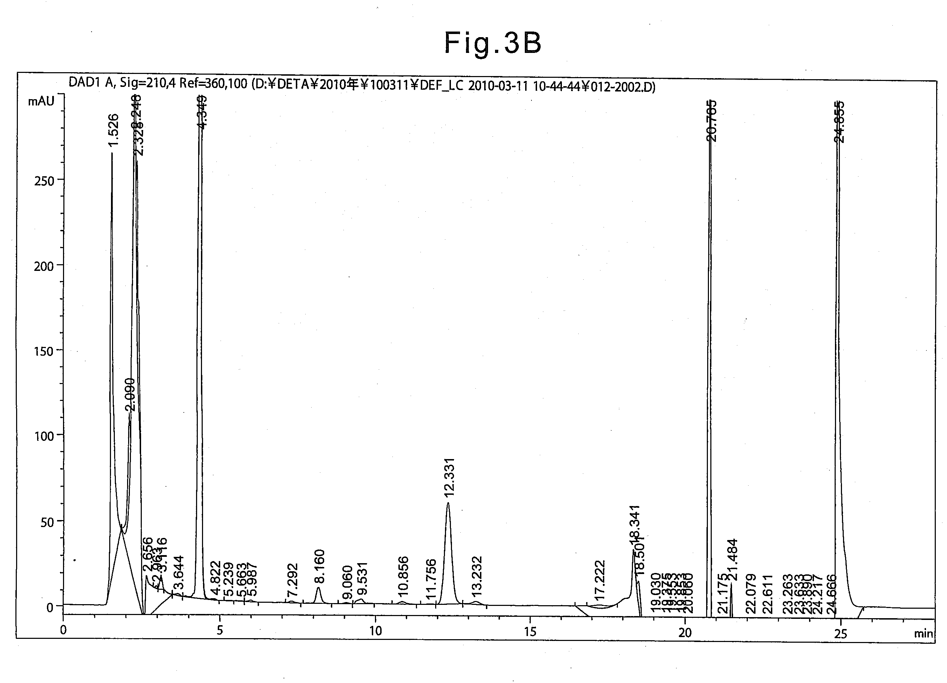

Allene oxide synthase derived from Lemna paucicostata SH strain of the present invention was shown to have a high activity compared to the conventionally used allene oxide synthase (Fig. 3). This high activity is probably due to the fact that this allene oxide synthase does not exhibit a suicide substrate-like property carried by a generally known allene oxide synthase. Thus, an α-ketol unsaturated fatty acid including KODA can be efficiently prepared in an enzymatic synthesis with α-linolenic acid or linoleic acid as the starting substance, by using the allene oxide synthase of the present invention. Since allene oxide synthase also contributes to the jasmonic acid biosynthetic pathway, it will allow to prepare jasmonic acid efficiently.

### [Brief Description of Drawings]

[Fig. 1]

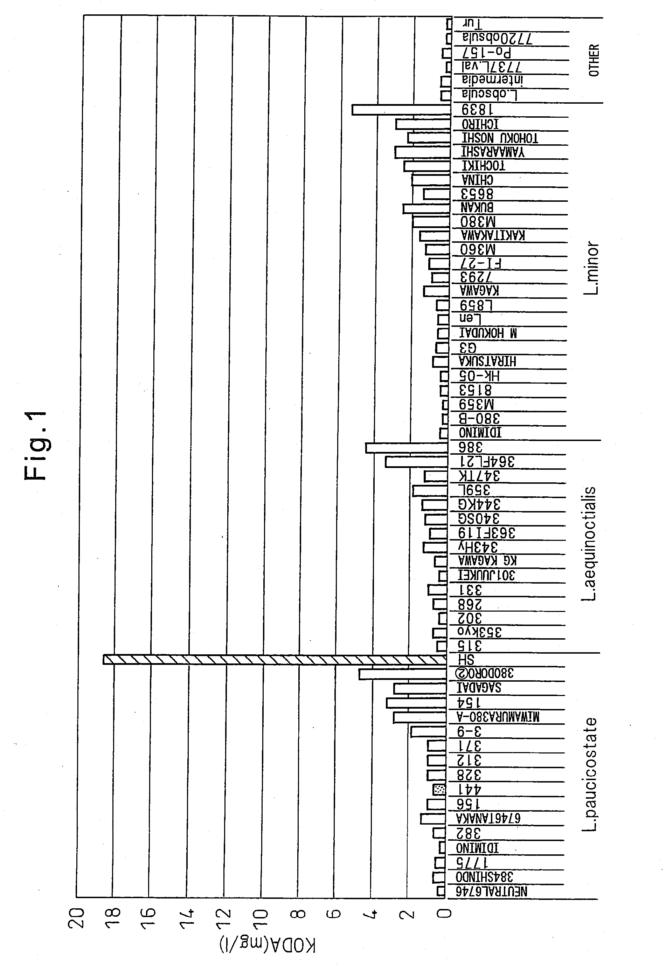

   Fig. 1 is a graph showing the amount of KODA produced for 62 strains of Lemna paucicostata.
[Fig. 2A]

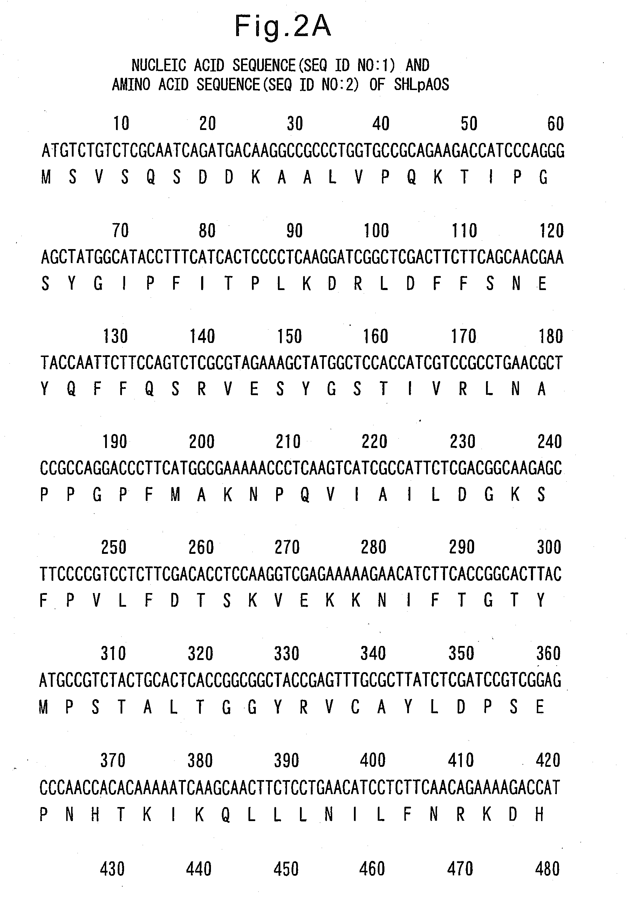

   Fig. 2A shows the nucleic acid sequence and amino acid sequence of the allene oxide synthase gene of Lemna paucicostata SH strain.
[Fig. 2B]

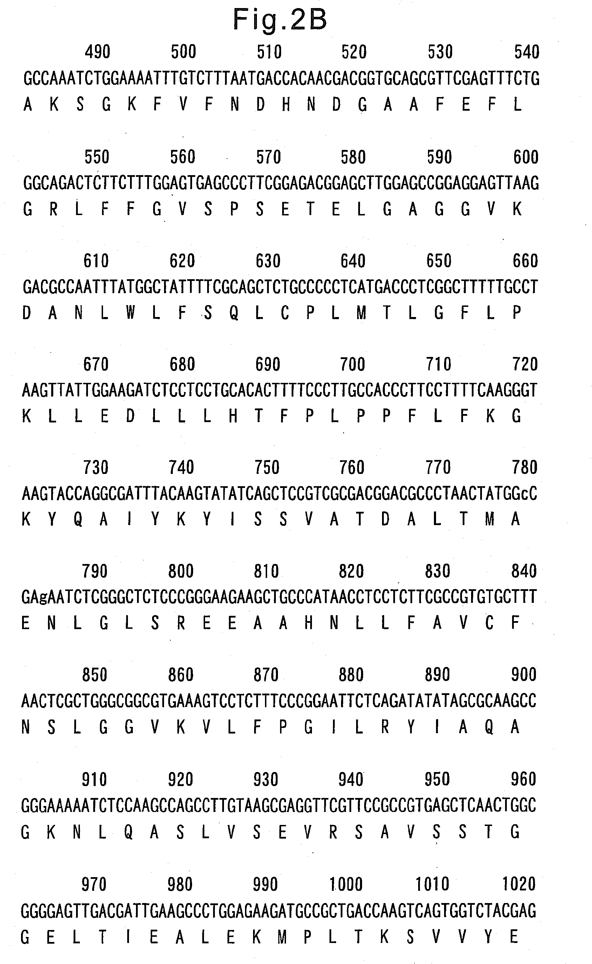

   Fig. 2B shows the continuation of the sequence of Fig. 2A.
[Fig. 2c]

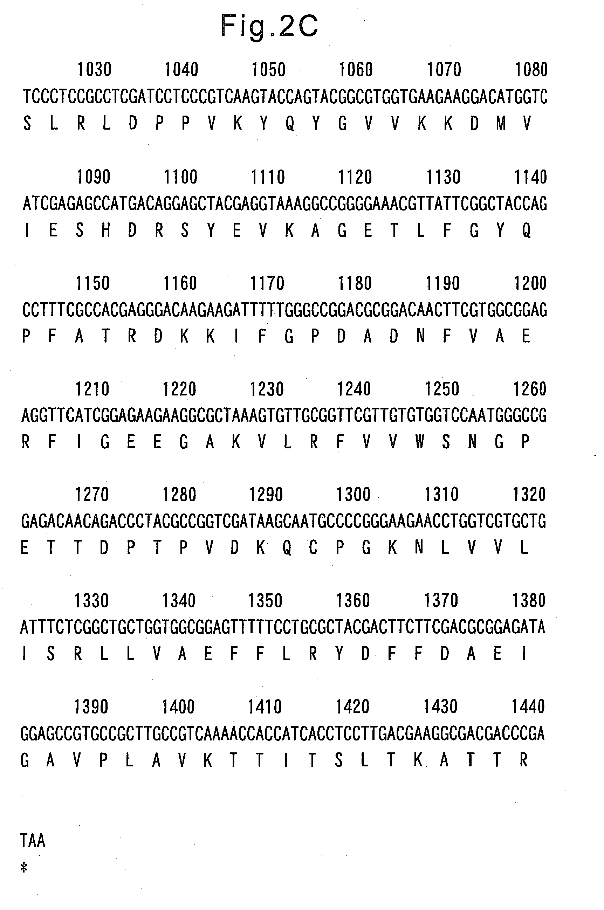

   Fig. 2C shows the continuation of the sequence of Fig. 2B.
[Fig. 3A]
   Fig. 3A shows the HPLC chromatogram of KODA formation using Arabidopsis thaliana AOS.
[Fig. 3B]
   Fig. 3B shows the HPLC chromatogram of KODA formation using SHLpAOS.
[Fig. 4]
   Fig. 4 shows the comparison of the activity of the allene oxide synthase (SHLpAOS) of Lemna paucicostata SH strain and the allene oxide synthase (AtAOS) of Arabidopsis thaliana used as the control, which are expressed in Escherichia coli.

### [Description of Embodiments]

The embodiments of the present invention are explained below. The present invention relates to a novel allene oxide synthase gene derived from Lemna paucicostata SH strain and a protein encoded by said gene. Specifically, the present invention relates to a gene consisting of a DNA having a base sequence of SEQ ID NO: 1 and a DNA substantially identical with said DNA. Furthermore, the present invention relates to a gene consisting of a DNA that encodes a protein consisting of an amino acid sequence of SEQ ID NO: 2, and a gene consisting of a DNA that encodes a protein consisting of an amino acid sequence substantially identical to said amino acid sequence. Furthermore, the present invention also relates to a fragment of the above DNA that encodes a protein having an allene oxide synthase activity.

As used herein the term "substantially identical DNA" refers to a DNA that has a 70% identity in the base sequence with the reference DNA. The identity may preferably be at least 80%, at least 90%, at least 95%, at least 97%, at least 98%, at least 99%, at least 99.5%, or at least 99.9%. Furthermore, "substantially identical DNA" also refers to a DNA that can hybridize under a high stringent condition to a DNA comprising a base sequence complementary to the reference DNA. The "substantially identical DNA", when transcribed and translated, has the same enzyme activity as that of a protein encoded by the reference DNA, i.e., the allene oxide synthase activity.

Hybridization can be carried out by a known method or a method based on said method, such as a method described in J. Sambrook et al., Molecular Cloning, 2nd., Cold Spring Harbor Laboratory Press, 1989. In this connection, a high stringent hybridization condition refers to, for example, a NaCl concentration of about 10-40 mM, preferably about 20 mM, and a temperature of about 50-70°C, preferably about 60-65°C.

The present invention also relates to a protein consisting of an amino acid sequence of SEQ ID NO: 2 and a protein consisting of an amino acid sequence substantially identical to said amino acid sequence. Furthermore, the present invention relates to a protein that is encoded by a DNA having a base sequence of SEQ ID NO: 1 or a DNA substantially identical to said DNA, and that has an allene oxide synthase activity. Furthermore, the present invention also relates to a fragment of said protein, having an allene oxide synthase activity.

As used herein "a protein comprising a substantially identical amino acid sequence" refers to a protein that comprises an amino acid sequence in which one or a few amino acids have been deleted, substituted or added in the amino acid sequence of the reference protein, and that has the activity of the reference protein, or an allene oxide synthase activity. Furthermore, "a protein comprising a substantially identical amino acid sequence" refers to a protein that comprises a sequence having an identity of at least 70% with the reference amino acid sequence and that has an allene oxide synthase activity. The identity may preferably be at least 80%, at least 90%, at least 95%, at least 97%, at least 98%, at least 99%, at least 99.5%, or at least 99.9%.

As used herein SEQ ID NO: 1 represents the base sequence of the allene oxide synthase gene derived from Lemna paucicostata SH strain, and SEQ ID NO: 2 represents the amino acid sequence of said allene oxide synthase. Specific sequences thereof are shown in Figs. 2A to C.

The allene oxide synthase activity means an enzyme activity of converting a hydroperoxidated fatty acid to an allene oxide.

As used herein allene oxide synthase or a protein having an allene oxide synthase activity refers to one that is recognized to have an allene oxide synthase activity by, for example, an activity determination method described below. For example, compared to the activity of allene oxide synthase having an amino acid sequence represented by SEQ ID NO: 2, those having an activity of at least 10%, at least 50%, at least 70%, at least 80%, at least 90%, more preferably at least 95%, and furthermore 100% or more, for example 110% or more, 110% or more, 120% or more, 150% or more, 200% or more are encompassed.

The gene (cDNA) of the present invention can be obtained by, for example, the following procedure. Primers are designed from an amino acid sequence common to a plant-derived allene oxide synthase, and RT-PCR is carried out to obtain a cDNA fragment. This fragment is labelled, as needed, with ³²P or digoxgenine, and using this in 5'-RACE or 3'-RACE, or using a cDNA library constructed in a standard method, screening can be carried out to obtain the full-length of the allene oxide synthase gene (cDNA). The determination of the base sequence can be conducted by a common method such as the Sanger method and the Maxam Gilbert method.

In an alternative method, Lemna paucicostata-derived allene oxide synthase is purified using ammonium sulfate fractionation or various chromatographic methods, separated by SDS polyacrylamide gel electrophoresis, electrically transferred to a PVDF membrane etc., and a band detected with a dye such as Coomassie brilliant blue is excised. Then, the N-terminal amino acid sequence is determined with a protein sequencer. Based on this sequence, primers are designed, and PCR is carried out according to the method mentioned above to obtain the full-length of the allene oxide synthase gene (cDNA).

The isolated gene (cDNA) can be inserted into a plasmid, virus etc., by a known method to prepare an expression vector, which is then introduced into the host cells such as a microorganism, the cultured cells of animal cells, plant cells, insect cells and is expressed, and thus the protein can be produced in large quantities. Furthermore, as the protein-expression system, a cell-free protein expression system, the cultured cells of a system using a wheat germ extract, may be used.

As a vector, an Escherichia coli-derived plasmid such as pBR322, pBR325, pUC18 and pUC119, a Bacillus subtilis-derived plasmid such as pUB110, pTP5 and pC194, a yeast-derived plasmid such as pSH19 and pSH15, a bacteriophage such as λ phage, an animal virus such as retrovirus, vaccinia virus and baculovirus, and pAl-11, pXT1, pRC/CMV, pRC/RSV, pcDNAI/Neo etc., can be used but not limited to them. As used herein a commercially available vector pET41a (Novagen) can be used in the the present invention.

The expression vector comprising the gene of the present invention can be produced by ligating the gene of the present invention downstream to a promoter of a suitable vector via a suitable restriction site by a known method. The promoter to be used in the present invention may not be specifically limited as long as it is a suitable promoter corresponding to the host to be used for the expression of the gene. For example, when an animal cell is used as the host, SRα promoter, SV40 promoter, LTR promoter, CMV promoter, HSV-TK promoter, β-actin promoter etc., can be mentioned. When the host is a bacteria of the genus Escherichia such as Escherichia coli, trp promoter, lac promoter, recA promoter, λPL promoter, lpp promoter, T7 promoter etc., may preferably be used, when the host is a bacteria of the genus bacillus, SPO1 promoter, SP02 promoter, pen promoter may preferably be used, and when the host is a yeast, PHO5 promoter, PGK promoter, GAP promoter, ADH promoter etc., may preferably be used. When the host is an insect, polyhedron promoter, P10 promoter etc., may be preferred. As desired, the expression vector may further contain an enhancer, a splicing signal, a polyA addition signal, a selection marker, SV40 replication origin, etc.

The host cell may be, but not limited to, a procaryotic cell or an eucaryotic cell that is conventionally used as the host for producing recombinant protein, and may include a mammal cell, an insect cell, a plant cell or a fungal cell, and preferably microorganisms such as bacteria or fungi. The term "fungus" is intended to encompass filamentous microorganisms and yeasts. Examples of suitable bacteria include Gram positive bacteria including genus Escherichia such as Escherichia coli, genus Bacillus such as Bacillus subtilis, and genus Streptomyces such as S. lividans, and Gram negative bacteria including genus Pseudomonas such as P. cepacia. The cells may be of fungi, i.e. yeast or filamentous cells. Yeast may be cells of genus Saccharomyces such as S. cereviceae. Filamentous host orgamisms may be, for example, Aspergillus strains such as A. niger. As insect cells, Sf cells, MG1 cells, BmN cells etc., may be mentioned. As animal cells, for example, monkey cell COS-7 cells, Vero cells, Chinese hamster cells CHO etc., may be mentioned. As used herein, Escherichia coli cells may specifically be preferred.

For the transformation of host cells, a known method such as the calcium chloride method, the calcium phosphate coprecipitation method, the DEAE dextran method, the lipofectin method, the protoplast polyethylene fusion method, the electroporation method etc., can be used, and may be selected as appropriate depending on the host cell to be used.

The protein of interest can be recovered by culturing the recombinant host cells obtained in a suitable culture medium, separating the cells from the culture liquid by a common procedure including, but not limited to, centrifugation or filtration, disrupting the cells as needed, precipitating the proteinaceous components of the supernatant or the filtrate by a salt such as ammonium sulfate, and then performing various chromatographic procedures such as ion exchange chromatography and an affinity chromatography. In order to attain the recovery of the protein of interest, a vector containing a tag sequence can be used. As the tag, His tag, GST tag, c-Myc tag, HA tag etc., can be mentioned.

By ligating a suitable signal sequence for secretion in microorganisms or animals upstream to DNA encoding the protein of the present invention, said protein can be secreted and expressed in the culture medium. Such a DNA modified to a secretary type is useful since it facilitates the purification of the protein secreted into the culture medium. As examples of such a signal sequence, there can be mentioned the pel B signal (S.P. Lei et al., J. Bacteriology, 169: 4379-4383, 1987) in the case of Escherichia coli, α factor signal (A.J. Brake, Yeast Genetic Engineering, p269 Butterworth, 1989) in the case of yeast, the signal SG-1 of immunoglobulin (H. Maeda et al., Hum. Antibod. Hybridomas, 2: 124-134, 1991), C25 signal (WO94/20632) etc., in the case of animal cells.

The allene oxide synthase activity can be determined by allowing a 9-product specific lipoxygenase to act on α-linolenic acid, and then adding a given amount of allene oxide synthase to the enzymatic reaction product 9-hydroperoxylinolenic acid (9-HPOT) to produce KODA, and the KODA produced is quantitatively analyzed by HPLC.

The protein of the present invention may be useful as a reagent for use in the synthesis of various linoleic acid and linolenic acid derivatives. Specifically, the protein of the present invention is useful as an enzyme to be used when an α-ketol unsaturated fatty acid, specifically KODA, useful as a plant growth regulating agent, is enzymatically synthesized from linoleic acid or linolenic acid. Also, it may be estimated that by introducing the gene of the present invention into a plant, the metabolism of linoleic acid or linolenic acid present in the plant by allene oxide synthase can be controlled.

The present invention will now be explained more specifically with reference to examples below. However, these examples do not limit the technical scope of the present invention in any way.

### Examples

### Example 1: Screening of a high KODA-producing Lemna paucicostata strain

62 types of Lemna paucicostata harvested from different places were prepared and were passaged in the 1/2-diluted Hutner's medium under continuous illumination from a 24-25°C daylight fluorescent light. The 1/2-diluted Hutner's medium had the following ingredients:

| | |
|---|---|
| Sucrose | 10 g/l |
| K₂HPO₄ | 200 mg/l |
| NH₄NO₃ | 100 mg/l |
| EDTA free acid | 250 mg/l |
| Ca(NO₃) · 4H₂O | 176 mg/l |
| MgSO₄ · 7H₂O | 250 mg/l |
| FeSO₄ · 7H₂O | 12.4 mg/l |
| MnCl₂ · 4H₂O | 8.92 mg/l |
| ZnSO₄ · 7H₂O | 32.8 mg/l |
| Na₂MoO₄ · 2H₂O | 12.6 mg/l |
| H₃BO₃ | 7.1 mg/l |
| Co(NO₃) · 6H₂O | 0.1 mg/l |
| CuSO₄ · 5H₂O | 1.97 mg/l |

and pH was adjusted to 6.2-6.5 with KOH (50%).

The grown Lemna paucicostata was unfolded on a filter paper, and after allowing to stand for 2 hours, it was immersed in water for 1 hour. For the water, the concentration of KODA was analyzed using high performance liquid chromatography (HPLC; column: TYPE UG120 5 µm, SIZE: 4.6 mm I.D.×250 mm; guard filter: INERTSTL 4.6mm×50 mm; eluent: 50% acetonitrile+0.1% trifluoroacetic acid; condition: absorption wavelength 210 λ(nm), flow rate: 1 ml/min, column temperature: 40°C). The mean of the amount produced of KODA in all Lemna paucicostata strains was 4.97 µM. Among them, Lemna paucicostata SH strain produced 60.2 µM of KODA, which is about 12-times as much as the mean amount of KODA produced, giving a very high production amount (Fig. 1).

### Example 2: Cloning of allene oxide synthase derived from Lemna paucicostata SH strain and its activity measurement

From Lemna paucicostata SH strain, total RNA was extracted using RNeasy Plant Mini Kit (QIAGEN), and then cDNA was synthesized using LongRange 2 Step RT-PCR Kit (QIAGEN) from 1.8 µg of total RNA as the template. Then, using the primers derived from Arabidopsis thaliana, and setting the annealing temperature for PCR at a low temperature of 45°C, a partial sequence information of allene oxide synthase derived from the SH strain (SHLpAOS) was obtained by using the synthesized cDNA as the template.
AOS-Forward: 5'-GGAACTAACCGGAGGCTACCG-3' (SEQ ID NO: 3)
AOS-Reverse: 5'-CCGTCTCCGGTCCATTCGACCACAA-3' (SEQ ID NO: 4)

Based on this sequence information, the full-length sequence was determined by the 3' or 5' RACE (Rapid Amplification of cDNA end) method.

### Primer for 3'-RACE

SH-3'-TP: 5'-TCTGGGCAGACTCTTCTTTGGAG-3' (SEQ ID NO: 5)

### Primer for 5'-RACE

SH-5'-TP: 5'-CCGGCTTGCGCTATATATCTGAGAATT-3' (SEQ ID NO: 6)

### 3'-RACE

By using 2 µg of total RNA of the SH strain obtained in the above experiment as the template, cDNA was synthesized using CapFishing^{™} target full-length cDNA cloning Kit (Seegene). PCR was carried out by using an addapter primer (3'-RACE primer) in the kit and SH-3'-TP, and this cDNA as the template solution.

### 5'-RACE

By using 2 µg of total RNA of the SH strain obtained in the above experiment as the template, cDNA was synthesized using CapFishing^{™} target full-length cDNA cloning Kit (Seegene). PCR was carried out by using an addapter primer (5'-RACE primer) in the kit and SH-5'-TP, and this cDNA as the template solution.

After the sequence of the PCR product was determined, PCR was performed by using the primers shown below and the above cDNA, and the entire sequence of cDNA was subcloned into pTAC-2 vector (BioDynamics). In order to insert into an expression vector, a NcoI site was added at the 5'-end and a XhoI site was added at the 3'-end.
SHAOS-F: 5'-CCGCGCCATGGAGAAGAGAATGTCTGTCTCGC-3' (SEQ ID NO: 7)
SHAOS-R: 5'-CCGCGCTCGAGGTTGAATAAGCACGAGTGT-3' (SEQ ID NO: 8)
As a result, a novel AOS homolog (nucleotide sequence: 1443 bp, amino acid sequence: 480 aa, deduced molecular weight: 53.3 KDa) of one sequence was obtained from SH strain (Figs. 2A-C). The allene oxide synthase obtained from the Lemna paucicostata SH strain was designated as SHLpAOS.

After the SHLpAOS sequence integrated into the pTAC-2 vector by the above method was cleaved with NcoI and XhoI, it was introduced into a commercially available vector pET41a (Novagen) for protein expression to obtain a plasmid pET41a/SHLpAOS for expression of Lemna paucicostata allene oxide synthase. This plasmid was transformed into Escherichia coli strain BL21(DE3) (Novagen). Then, it was inoculated in 3 ml of the LB medium containing 30 mg/l kanamycin, and cultured under shaking overnight at 37°C and 250 rpm. 100 µl of this culture liquid was added to 10 ml of the LB medium containing the same concentration of kanamycin and 1% glucose and cultured under shaking at 37°C and 250 rpm for 3-4 hours. From 25 ml of this culture liquid, bacterial body were recovered by centrifugation, transferred to 50 ml of the TB medium containing 0.1 mM isopropyl-β-D-thiogalacto-pyranoside (Wako Pure Chemical Industries, Ltd.) and 30 mg/l of kanamycin, and cultured overnight under shaking at 25°C and 250 rpm to induce allene oxide synthase. After culturing was over, centrifugation was carried out, and 5 ml of BugBuster^{™} (Novagen) was added to 1 g of the wet bacterial body mass to dissolve the bacteria. Then, centrifugation was carried out (9,000 rpm, 5 minutes, 4°C), and the supernatant was recovered to prepare an enzyme solution. Using this enzyme solution, the activity testing in KODA production was carried out.

In the activity testing in KODA production, 5 mM linolenic acid solution (dissolved in 0.1% Tween 80 solution) was prepared, and reacted with lipoxygenase extracted from rice germ at pH 7 at room temperature for 10 minutes to synthesize 9-hydroperoxylinolenic acid (9-HPOT) solution. To 20 µl of this 9-HPOT reaction solution, 0.32 ng of the SHLpAOS protein or A. thaliana-derived AOS (AtAOS) protein was added and reacted at room temperature for 10 minutes. After the reaction was over, the reaction was terminated by a heat treatment at 50°C for 3 minutes. 10 µl of this solution was analyzed by HPLC. HPLC analysis was carried out with a column: Capsule pack C-18 UG120 (4.6×250 mm, Shiseido), column temperature: 40°C, mobile phase: 50% acetonitrile solution (0.02% TFA), flow rate: 1 ml/min, detection wavelength: 210 nm. The result of the HPLC is shown in Fig. 3 (A, B). As can be seen from the HPLC chart, the peak of KODA was confirmed indicating that allene oxide synthase derived from Lemna paucicostata SH strain can be used in KODA production. Also, the SHLpAOS protein had an activity nearly 7-fold higher than the AtAOS protein (Fig. 4).

SHLpAOS obtained from Lemna paucicostata SH strain was subjected to kinetic analysis. Using a reaction mixture comprising 40 mM phosphate buffer (pH 7.5) and 1% EtOH, the reaction was carried out at a temperature of 25°C. The substrate 9-HPOT was tested at the substrate concentration of 5-53 µM. The substrate 9-HOPT was added as an EtOH solution, and the final concentration of EtOH was adjusted to 1%. 100 µl of the reaction mixture was added into a cuvette, and a decrease in absorbance at 234 nm was scanned over time for 1 minute at an interval of 2 seconds using the SmartSpec Plus Spectrophotometer (Bio-Rad). From the A₂₃₄ determined, the amount of the reaction product was calculated (e=25,000). Kinetic parameters were determined using the Hanes-Woolf plot ([S]/v versus [S] plot). The result indicates that in SHLpAOS the Kₘ value is significantly lower than AtAOS and had an about 5-fold higher affinity for 9-HPOT. The Vₘₐₓ was about 2.8-fold higher than AtAOS. The K_{cat} value was also about 2.8-fold higher in SHLpAOS than in AtAOS, indicating that reaction turnover is occurring very efficiently. The K_{cat}/Kₘ value was about 14-fold higher in SHLpAOS than in AtAOS. It is believed that compared to AtAOS, SHLpAOS is a very useful AOS in the practical production of KODA. The above revealed that SHLpAOS cloned from Lemna paucicostata SH strain is an AOS having a very high activity that has not been reported before.

**[Table 1] Kinetic parameters of SHLpLOX and AtAOS**

| | Kₘ (µM) | Vₘₐₓ (µmol sec⁻¹) | K_{cat} (sec⁻¹) | K_{cat}/Kₘ (×10⁶M⁻¹sec⁻¹) |
|---|---|---|---|---|
| LpLOX-SH | 7.1±1.9 | 4.7±0.3 | 709.2±44.3 | 99.0±23.7 |
| AtAOS | 36.3±1.3 | 1.7±0.03 | 255.5±4.8 | 7.0±3.8 |

## Claims

1. A gene consisting of DNA selected from the group consisting of the following (a) to (e):
(a) a DNA comprising a base sequence of SEQ ID NO: 1;
(b) a DNA that encodes a protein consisting of an amino acid sequence of SEQ ID NO: 2;
(c) a DNA encoding a protein that comprises an amino acid sequence in which one or a few amino acids have been deleted, substituted or added to an amino acid sequence of SEQ ID NO: 2, and that has an allene oxide synthase activity;
(d) a DNA that is at least 90% identical to DNA consisting of a base sequence of SEQ ID NO: 1 and that encodes a protein having an allene oxide synthase activity; and
(e) a DNA that hybridizes under a high stringent condition to a DNA consisting of a base sequence complementary to a DNA consisting of a base sequence of SEQ ID NO: 1 and that encodes a protein having an allene oxide synthase activity.

2. An expression vector comprising the DNA according to claim 1 and a vector fragment bound to each other therein.

3. A transformant wherein the expression vector according to claim 2 has been introduced into a host microorganism, animal cell or plant cell.

4. A method of producing allene oxide synthase which comprises culturing the transformant according to claim 3.

5. A protein selected from the group consisting of the following (a) to (d):
(a) a protein consisting of an amino acid sequence of SEQ ID NO: 2;
(b) a protein that consists of an amino acid sequence in which one or a few amino acids have been deleted, substituted or added to an amino acid sequence of SEQ ID NO: 2, and that has an allene oxide synthase activity;
(c) a protein that is encoded by a DNA that is at least 90% identical to a DNA consisting of a base sequence of SEQ ID NO: 1 and that has an allene oxide synthase activity; and
(d) a protein that is encoded by a DNA capable of hybridizing under a high stringent condition to a DNA consisting of a base sequence complementary to a base sequence of SEQ ID NO: 1 and that has an allene oxide synthase activity.
